# EUROPEAN PATENT SPECIFICATION

(11) **EP 2 600 849 B1**
(45) Date of publication and mention of the grant of the patent: **30.03.2016**
(21) Application number: 10855698.6
(22) Date of filing: 04.08.2010
(51) Int. Cl.: A61K 9/48

(54) **FILM-FORMING COMPOSITION FOR SOFT CAPSULES**
FILMBILDENDE ZUSAMMENSETZUNG FÜR WEICHE KAPSELN
COMPOSITION FORMANT UN FILM POUR CAPSULES MOLLES

(43) Date of publication of application: 12.06.2013
(73) Proprietor: R.P. Scherer Technologies, LLC, Las Vegas, Nevada 89119 (US)
(72) Inventor: FUJII, Takuma, Kakegawa City Shizuoka Prefecture 436-0028 (JP); NAGURA, Masanori, Fukuroi City Shizuoka Prefecture 437-0023 (JP); AMEMIYA, Tohru, Kakegawa City Shizuoka Prefecture 436-0022 (JP)
(74) Representative: Dunleavy, Kevin James
(86) International application number: PCT/US2010/044419
(87) International publication number: WO 2012/018329

(56) References cited:
- EP-A1- 1 598 062
- WO-A2-01/91721
- WO-A2-01/92400
- US-A1- 2005 196 436
- US-A1- 2006 099 246
- US-A1- 2008 138 402
- US-B1- 6 214 376
- US-B1- 6 375 981

## Description

### Technical Field

The present invention relates to a film-forming composition for soft capsules suitably used for forming soft capsules and a soft capsule prepared using the composition.

### Background

Soft capsules have widely been employed in the fields of pharmaceutical preparations, cosmetics and foods. In this respect, gelatin has widely been used as a principal component for constituting the shell of such a soft capsule, and gelatin is mainly produced from the bone of cattle and the skin of swine. However, a problem arises such that vegetarians, people of Islamic faith who cannot eat swine, and those of the Hindu faith who believe that cattle are sacred and inviolable, cannot consume a soft capsule containing such gelatin derived from cattle and/or swine.

Moreover, with respect to gelatin produced from the bone of cattle, there is a perception that there is the possibility of infection with bovine spongiform encephalopathy. Also, gelatin, other than that derived from cattle and swine, suffers from problems such that the gelatin does not provide the desired strength required for forming a gelatin capsule and it may increase the production cost of the gelatin capsule as its cost is generally higher when compared with the gelatin derived from cattle and swine.

On the other hand, there have been known vegetable soft capsules whose shells are formed without using gelatin (see, for instance, JP 2003-504326A, JP 2008-519075A, and JP 2005-176744A). Such vegetable soft capsules are produced from components derived from plants and therefore, they never suffer from a problem attributable to the use of the gelatin derived from cattle and swine. Soft capsules prepared using gelatin, are in general insolubilized with the lapse of time resulting in slower disintegration of the shell and slower release or in some circumstances a failure to release the capsule contents. Contrary to this, the vegetable soft capsule has an advantage in that it sparingly causes any insolubilization with the lapse of time and has good physical stability. However, it was found that such a vegetable soft capsule suffers from a problem such that the workability or processability of the material derived from plants encountered when it is formed into a capsule is insufficient and that the strength of the shell produced from the same is likewise unsatisfactory. Additional prior art includes US 2006/099246.

More specifically, unmodified starch (such as rice starch and corn starch) suffers from a problem in that it has a high viscosity, and it would be difficult to blend the starch with a gelling agent and/or a plasticizer and that the workability thereof upon the production of capsules is accordingly impaired. In this connection, the strength of a capsule, prepared using corn starch which has been decomposed even to dextrin according to the enzyme treatment as one of the physical treatments to reduce the viscosity, is quite low and accordingly, the resulting product (capsule) cannot be used. It further suffers from a problem in that the strength thereof is extremely reduced with the lapse of time.

For this reason, the development of a vegetable capsule which possesses excellent characteristics both in the preparation of a soft capsule and in subsequent storage is very desirable.

### SUMMARY OF THE INVENTION

It is an object of the present invention to provide a film-forming composition for the preparation of a soft capsule which mainly comprises a component other than gelatin.

### DETAILED DESCRIPTION

The present invention has been completed on the basis of such a finding that acid-decomposed waxy corn starch, among a variety of starches, is suitable as a substitute for gelatin and that when combining waxy corn starch with a gelling agent and a plasticizer, a shell can be formed, which has excellent characteristic properties suitable for use in the preparation of a soft capsule.

More specifically, the present invention provides a film-forming composition for use in the preparation of a soft capsule, which comprises (a) acid-decomposed waxy corn starch; (b) a gelling agent; and (c) a plasticizer.

The present invention also provides a soft capsule which comprises a shell formed from the foregoing composition and a capsule-filling material accommodated within the shell.

The present invention can provide a shell for soft capsules, which is completely free of any material derived from animals such as gelatin and a soft capsule which comprises the shell and capsule-filling material accommodated within the shell. The soft capsule comprising the shell produced using the film-forming composition for soft capsules has excellent characteristic properties with respect to physical strength, disintegration ability, odor, taste, color and lack of stickiness or adhesion; it also has excellent stability with the lapse of time. In addition, the use of, in particular, carrageenan as the gelling agent (b) would have an advantage in that a film having a strength sufficient for processing and forming into a capsule can be formed without using any metal salt (such as a sodium salt, a potassium salt or a calcium salt), which salts are in general required for the gelatinization of carrageenan.

Corn can be divided into species of dent corn and waxy corn, but the starch derived from the corn belonging to the waxy corn species is the objective starch to be used in the present invention as the component (a). The corn of the waxy species is also referred to as glutinous corn, and the starch derived therefrom is generally one which is free of any amylase and substantially comprises 100% amylopectin. The acid-decomposed waxy corn starch used in the present invention includes acid hydrolyzed waxy corn starch and acid roasted or broiled waxy corn starch. The acid-decomposed waxy corn starch suitable for use in the present invention can be made according to known processes or purchased from commercial sources. The acid-decomposed waxy corn starch is, for instance, one obtained by adding an inorganic acid or an organic acid such as sulfuric acid, or an oxidizing agent such as sodium hypochlorite to the starch derived from the corn of the waxy species and then heating the resulting mixture at a temperature ranging from about 10 to about 160°C to thus partially decompose the starch structure. Among those preferably used in the present invention are those having a content of carboxyl groups on the order of not higher than 0.1 % by mass. In addition, preferably used herein are those each having a viscosity ranging from about 5 to about 100 mPa • S, as determined at 80°C using a 20% by mass aqueous solution thereof. The viscosity of the starch can be determined using a BM viscometer (VISCO-BM Model, available from TOKIMECH Co., Ltd.) and a rotor no. 1. The acid-decomposed waxy corn starch is commercially available as, for instance, WS-10 (available from MATSUTANI Chemical Industry, Co., Ltd.).

Particularly preferred acid-decomposed waxy corn starches include those having a viscosity ranging from about 5 to about 50 mPa • S and more preferably about 10 to about 20 mPa • S, as determined under the foregoing conditions.

The amount of the acid-decomposed waxy corn starch as the component (a) used in the film-forming composition for soft capsules according to the present invention preferably ranges from about 10 to about 60% by mass and more preferably about 25 to about 60% by mass (on the basis of the dry mass of the composition or the mass thereof except for water).

Examples of the gelling agents as the component (b) used in the present invention are carrageenan (τ- carrageenan, κ-carrageenan and λ-carrageenan), agar, gum arabic, gellan gum, native gellan gum, pullulan, pectin, glucomannan, locust bean gum, guar gum, geran gum, cellulose, konjak-derived gum, furcellaran, tara-derived (Japanese angelica tree-derived) gum, alginic acid and tamarind gum, which may be used alone or in any combination of at least two of them. Among them, preferably used herein are carrageenan (t- carrageenan, κ-carrageenan and λ-carrageenan), agar, gum arabic, gellan gum, native gellan gum, pullulan, pectin and glucomannan, which may be used alone or in any combination of at least two of them. Particularly preferably used herein as the gelling agents are τ- carrageenan, κ-carrageenan and mixtures thereof, among others.

The amount of the gelling agent used as the component (b) of the film-forming composition for soft capsules according to the present invention preferably ranges from about 8 to about 30% by mass and more preferably about 10 to about 24% by mass (on the basis of the dry mass of the composition or the mass thereof except for water).

Examples of the plasticizers used in the composition of the present invention as the component (c) include glycerin, sorbitol, maltitol, propylene glycol, polyethylene glycol, sugar alcohol, lactitol, polyalkylene glycol, monosaccharides, disaccharides, oligosaccharides, isomalto-oligosaccharides, diethylene glycol, glycerol monoacetate, glycerol diacetate, glycerol triacetate, invert sugars, corn syrup and 1,2-propylene glycol, which may be used alone or in any combination of at least two of them. Among them, preferably used herein include glycerin, sorbitol, maltitol, propylene glycol, polyethylene glycol, sugar alcohol, lactitol, polyalkylene glycol, monosaccharides, disaccharides, oligosaccharides and isomalto-oligo- saccharides, which may be used alone or in any combination of at least two of them. Particularly preferably used herein as the plasticizer (c) are glycerin, sorbitol, polyethylene glycol and mixtures thereof.

The amount of the plasticizer as the component (c) used in the film-forming composition for soft capsules according to the present invention preferably ranges from about 5 to about 65% by mass, more preferably about 10 to about 60% by mass and particularly preferably about 15 to about 50% by mass (on the basis of the dry mass of the composition or the mass thereof except for water).

The film-forming composition for soft capsules according to the present invention may further comprise a buffering agent (d). Examples of such buffering agents are sodium salts, potassium salts and calcium salts, and the buffering agent (d) used in the invention is preferably sodium phosphate.

The amount of the buffering agent as the component (d) used in the film-forming composition for soft capsules according to the present invention preferably ranges from about 0.2 to about 5% by mass and more preferably about 1 to about 4% by mass (on the basis of the dry mass of the composition or the mass thereof except for water).

In the present invention, however, carrageenan is particularly preferably used as the gelling agent or the component (b) since this would permit the elimination of the need for any buffering agent such as sodium salts, potassium salts and calcium salts, which are in general required for the gelatinization of carrageenan. In this case, it is preferred to use the acid-decomposed waxy corn starch (a) and the gelling agent (b) in a ratio [(a)/(b)] (by mass) ranging from about 1/1 to about 4/1.

In addition, the film-forming composition for soft capsules according to the present invention may further comprise, as optional components, other additives, for instance, a coloring agent such as a coloring dyestuff or a pigment, a perfume and/or a preservative. In this respect, however, it is preferred that the composition is completely free of gelatin.

When forming a shell for soft capsules using the film-forming composition for soft capsules according to the present invention, it is suitable that water is added to and blended with the film-forming composition for soft capsules in an amount ranging from about 40 to about 110 parts by mass per 100 parts by mass of the film-forming composition, and the resulting blend is then formed into a shell according to the usual method in such a manner that the thickness of the shell is set at a level ranging from about 0.1 to about 1.0 mm. In this respect, it is also possible to form a seamless capsule with the use of the film-forming composition for soft capsules according to the present invention. Methods of making soft capsules are conventional and can readily be used with the film-forming composition of the present invention.

The soft capsule of the present invention may be filled with, for instance, a suspension or an oil as a capsule-filling material. One of ordinary skill in the art can readily determine a suitable fill material.

The present invention will hereunder be described in more detail with reference to the following examples and comparative examples, but the present invention is not restricted to these specific examples at all.

### EXAMPLES

In the following Table 1, there are listed starches and the components related thereto, which are used in the following examples and comparative examples.

**Table 1:**

| Product Name | Treating Method | Source Material | Classification |
|---|---|---|---|
| WS-10 | Acid-decomposition | Waxy corn | |
| Cluster dextrin | Enzyme-treatment | Waxy corn | Highly branched cyclic dextrin |
| MAX 1000 | Enzyme-treatment | Tapioca starch | Dextrin |
| Pinedex #100 | Enzyme-treatment | Waxy corn | Dextrin |
| WR-1 | Unprocessed | Rice starch | Starch |
| Purified, dried and sterilized corn starch | Unprocessed | Corn starch | Starch |

These starch products are all commercially available from MATSUTANI Chemical Industry Co., Ltd., Ezaki Glico Co., Ltd. and Nippon Corn Starch Mfg. Co., Ltd.

The viscosity of the acid-decomposed waxy corn starch WS-10 was determined according to the following method:

To 80 g of starch (WS-10), there was added 320 g of pure water; the mixture was immersed in a warm water bath maintained at 80°C to thus dissolve the starch in water and to obtain a 20% aqueous starch solution. The temperature of the starch was maintained at 80°C, while the solution was still immersed in the warm water bath and the viscosity was determined by the use of a BM viscometer (VISCO-BM Model available from TOKIMECH Co., Ltd. connected to a rotor no. 1. As a result, the viscosity thereof was found to be 12 to 16 mPa • S.

### Example 1

To the film-forming composition for soft capsules having the composition specified in the following Table 2, there was added pure water in an amount ranging from 40 to 110 parts by mass per 100 parts by mass of the composition to adjust the viscosity of the solution to a level sufficient for forming a capsule. Then a soft capsule was prepared from the resulting blend using a rotary die type encapsulation machine, while filling the resulting capsule with rapeseed oil as a capsule-filling material.

**Table 2**

| Component | Rate (%) |
|---|---|
| Acid-decomposed waxy corn starch (WS-10) | 46.9 |
| τ-Carrageenan | 15.2 |
| Glycerin | 36.5 |
| Sodium phosphate | 1.4 |

### Comparative Examples 1 and 2

In these Comparative Examples 1 and 2, the same procedures used in Example 1 were repeated except that rice starch (WR-1) and corn starch (purified, dried and sterilized corn starch) were, respectively, substituted for the acid-decomposed waxy corn starch (WS-10) used in the composition of Example 1 having a composition as specified in Table 2 to thus form a capsule. As a result, it was found that even when adding 40 to 110 parts of pure water, the compositions of Comparative Examples 1 and 2 had extremely high viscosities to such an extent that the machinery and tools for the preparation thereof were almost broken and that these compositions never permitted the preparation of any film-forming liquid.

### Comparative Examples 3 to 5

In these Comparative Examples 3 to 5, the same procedures used in Example 1 were repeated except that cluster dextrin (derived from waxy corn; Comparative Example 3), enzyme-treated dextrin (Pinedex #100; Comparative Example 4) and enzyme-treated dextrin (MAX 1000; Comparative Example 5) were, respectively, substituted for the acid-decomposed waxy corn starch (WS-10) used in the composition of Example 1 whose composition was specified in Table 2 to thus form a capsule.

The capsules produced in Example 1 and Comparative Examples 3 to 5 were inspected for the film strength and the stability thereof with the lapse of time, according to the following methods.

### Burst Test

Using a burst test machine specified below, a varying load is applied to one capsule to thus determine the load observed when the capsule is just cracked and the load is expressed in the unit kg:
Capsule Size: 9.5 Oblong;
Film Thickness: About 0.5 mm (0.4 to 0.6 mm);
Burst Test Machine: KIYA Type Hardness Meter (1600-E Type, available from Fujiwara Seisakusho, Ltd.)

### Disintegration Ability

This physical property was determined according to the disintegration testing method as specified in Japanese Pharmacopoeia, the 14^{th} revised edition. This is expressed in terms of the time period required for initiating the release of the charged medical fluid out of the capsule and that required for the complete dissolution of the capsule film.

The results are summarized in the following Table 3:

**Table 3**

| Ex. No. | | Ex. 1 | Comp. Ex. 3 | Comp. Ex. 4 | Comp. Ex. 5 |
|---|---|---|---|---|---|
| Breakage (kg) | | 23.8 | 14.8 | 15.1 | 20.8 |
| Disintegration Time (min) | Opening of the capsule | 2-3 | 0-4 | 1-3 | 3-4 |
| | Disintegration | 4-6 | 3-5 | 2-5 | 5-7 |
| | Dissolution of Film | 9-12 | 9-11 | 8-12 | 9-12 |

The results listed in the foregoing Table 3 clearly indicate that the soft capsule (Example 1) produced using the film-forming composition for soft capsules according to the present invention has a very high physical strength as determined in the burst test as compared with those observed for the capsules prepared in Comparative Examples 3 to 5, but there is not observed any substantial difference in the disintegration time between the capsule prepared in Example 1 and those prepared in Comparative Examples 3 to 5.

Moreover, the same tests were carried out after each of the capsules was stored at 40°C over 3 months. The results obtained are summarized in the following Table 4. The data listed in Table 4 clearly indicate that the capsule of the present invention still maintains its excellent physical strength and disintegration time.

The capsules prepared in Comparative Examples 3 to 5 showed the deterioration of the physical strength after the storage thereof at 40°C, while the capsule prepared in Example 1 did not show any deterioration of the physical strength even after the storage thereof at 40°C.

**Table 4**

| Samples were stored at 40°C for 3 months. | | | | | |
|---|---|---|---|---|---|
| Ex. No. | | Ex. 1 | Comp. Ex. 3 | Comp. Ex. 4 | Comp. Ex. 5 |
| Breakage (kg) | | 25.8 | 8.1 | 14.9 | 14.7 |
| Disintegration Time (min) | Opening of the capsule | 2-3 | 0-4 | 1-3 | 3-6 |
| | Disintegration | 4-6 | 4-5 | 3-4 | 5-7 |
| | Dissolution of Film | 9-12 | 9-12 | 8-11 | 9-12 |

In addition, the capsules produced in Example 1 and Comparative Examples 3 to 5 were inspected for the characteristic properties such as the appearance of the soft capsules. The results thus obtained are summarized in the following Table 5.

**Table 5: Quality of Capsules**

| Ex. No. | Ex. 1 | Comp. Ex. 3 | Comp. Ex. 4 | Comp. Ex. 5 |
|---|---|---|---|---|
| Odor of caramel | Not detected | Strong | Weak | Weak |
| Taste | Tasteless | Sweet | Slightly sweet | Slightly sweet |
| Color | Bright | Transparent, rather dark | Dark | Dark |
| Clumping of capsules due to stickiness | Weak | Weak | Weak | Strong |

As will be seen from the data listed in Table 5, the soft capsule (Example 1) produced using the film-forming composition for soft capsules according to the present invention was found to be excellent in all of the properties tested, or the odor, taste, color and lack of clumping, while the capsule prepared in Comparative Example 3 gives out a strong odor of caramel and has a taste of caramel. Accordingly, a problem arises such that the odor of caramel may impair the palatability of the capsule when eating the same. The capsule prepared in Comparative Example 4 suffers from such a problem that it has an insufficient transparency and appears to be dark, although it has weak odor and taste of caramel. The capsule prepared in Comparative Example 5 has an insufficient transparency, appears to be dark. Further the capsules prepared in Comparative Example 5 are mutually adhered to one another strongly, causing clumping and a problem thus arises when packaging the same.

### Example 2

The same procedures used in Example 1 were repeated except for using the composition specified in the following Table 6, or the film-forming composition for soft capsules used in Example 1, from which sodium phosphate as a buffering agent was removed, to thus form a soft capsule.

**Table 6**

| Component | Rate (part by mass) |
|---|---|
| Acid-decomposed waxy corn starch (WS-10) | 47.6 |
| τ-Carrageenan | 15.4 |
| Glycerin | 37.0 |

The soft capsule prepared in Example 2 was inspected for the physical strength. The results thus obtained are summarized in the following Table 7 together with those observed for the soft capsule prepared in Example 1, for the purpose of comparison.

**Table 7**

| Ex. No. | | Example 2 | Example 1 |
|---|---|---|---|
| | | Free of Sodium phosphate | Containing Sodium phosphate |
| Breakage (kg) | | 24.3 | 23.8 |
| Disintegration Time (min) | Opening of the capsule | 0.75 -3 | 2-3 |
| | Disintegration | 6-7 | 4-6 |
| | Dissolution of Film | 13-15 | 9-12 |

As will be seen from the data listed in Table 7, the present invention permits the production of a soft capsule with excellent physical strength without using sodium phosphate as a buffering agent. Moreover, when comparing the results listed in Table 3 with those listed in Table 7, it was found that the capsule prepared in Example 2 has a shorter opening time than that observed for the capsule prepared in Example 1, and the former has an excellent disintegration rate, which is one of the advantages of the soft capsule.

## Claims

1. A film-forming composition for use in the preparation of a soft capsule, said film-forming composition comprising:
(a) acid-decomposed waxy corn starch;
(b) a gelling agent; and
(c) a plasticizer.

2. The composition as set forth in claim 1, wherein the acid-decomposed waxy corn starch has a viscosity ranging from about 5 to about 100 mPa • S as determined using a 20% by mass aqueous solution thereof at 80°C.

3. The composition as set forth in claim 1, wherein the gelling agent is carrageenan.

4. The composition as set forth in claim 1, wherein the plasticizer is glycerin, sorbitol, polyethylene glycol or a mixture thereof.

5. The composition as set forth in claim 1, further comprising (d) a buffering agent.

6. The composition as set forth in claim 5, wherein the buffering agent is a sodium salt, a potassium salt or a calcium salt.

7. The composition as set forth in claim 1, wherein the composition is free of any buffering agent.

8. A soft capsule shell made using the film-forming composition of claim 1.

9. A soft capsule comprising the shell of claim 8 and a capsule-filling material accommodated within the shell.

## Patentansprüche

1. Filmbildende Zusammensetzung zur Verwendung bei der Herstellung einer Weichkapsel, wobei diese filmbildende Zusammensetzung wie folgt umfasst:
(a) Säurezerlegte, wächserne Maisstärke; ein
(b) Geliermittel und einen
(c) Weichmacher.

2. Zusammensetzung nach Anspruch 1, wobei die säurzerlegte, wächserne Maisstärke jeweils nach Bestimmung mittels einer wässrigen Lösung derselben von 20 Gew% bei 80 °C eine Viskosität im Bereich zwischen 5 bis 100 mPa • S aufweist.

3. Zusammensetzung nach Anspruch 1, wobei das Geliermittel Carrageen ist.

4. Zusammensetzung nach Anspruch 1, wobei der Weichmacher Glycerin, Sorbitol, Polyethylenglycol oder ein Gemisch davon ist.

5. Zusammensetzung nach Anspruch 1, wobei dieselbe ferner einen (d) Puffer aufweist.

6. Zusammensetzung nach Anspruch 5, wobei der Puffer ein Natriumsalz, Kaliumsalz oder Kalziumsalz ist.

7. Zusammensetzung nach Anspruch 1, wobei die Zusammensetzung pufferfrei ist.

8. Weichkapselschale, die aus der filmbildenden Zusammensetzung nach Anspruch 1 hergestellt wird.

9. Weichkapsel, die die Schale nach Anspruch 8 und ein die Kapsel befüllendes Material, das innerhalb der Schale untergebracht wird, umfasst.

## Revendications

1. Une composition formant un film destinée à être utilisée dans la préparation d'une capsule molle, la dite composition formant un film comprenant :
(a) de l'amidon de maïs cireux décomposé à l'acide ;
(b) un agent gélifiant ; et
(c) un plastifiant.

2. La composition comme décrite dans la revendication 1, dans laquelle l'amidon de maïs cireux décomposé à l'acide a une viscosité allant d'environ 5 mPa · S à environ 100 mPa · S déterminé par l'utilisation d'une solution aqueuse à 20% de masse de celle-ci à 80°C.

3. La composition comme décrite dans la revendication 1, dans laquelle l'agent gélifiant est du carraghénane.

4. La composition comme décrite dans la revendication 1, dans laquelle le plastifiant est de la glycérine, du sorbitol, du polyéthylène glycol ou un mélange de ceux-ci.

5. La composition comme décrite dans la revendication 1, comprenant de plus (d) un agent tampon.

6. La composition comme décrite dans la revendication 5, dans laquelle l'agent tampon est un sel de sodium, un sel de potassium ou un sel de calcium.

7. La composition comme décrite dans la revendication 1, dans laquelle la composition ne contient aucun agent tampon.

8. Une enveloppe de capsule molle fabriquée avec la composition formant un film de la revendication 1.

9. Une capsule molle comprenant l'enveloppe de la revendication 8 et un matériau de remplissage de capsule contenu à l'intérieur de l'enveloppe.
